# EUROPEAN PATENT SPECIFICATION

(11) **EP 2 263 079 B1**
(45) Date of publication and mention of the grant of the patent: **08.01.2020**
(21) Application number: 08745156.3
(22) Date of filing: 04.04.2008
(51) Int. Cl.: G01N 27/42, G01N 33/18

(54) **SIMULTANEOUS ELECTROCHEMICAL DETECTION OF MULTIPLE HEAVY METAL IONS IN LIQUID**
GLEICHZEITIGER ELEKTROCHEMISCHER NACHWEIS MEHRERER SCHWERMETALLIONEN IN FLÜSSIGKEIT
DÉTECTION ÉLECTROCHIMIQUE SIMULTANÉE DE PLUSIEURS IONS DE MÉTAUX LOURDS DANS UN LIQUIDE

(43) Date of publication of application: 22.12.2010
(73) Proprietor: Arizona Board of Regents on behalf of Arizona State University, Scottsdale, AZ 85257-9908 (US); EMD Millipore Corporation, Burlington, MA 01803 (US)
(72) Inventor: DIMITRAKOPOULOS, Telis, 28130 Saint Martin de Nigelles (FR); WANG, Joseph, Scottsdale, Arizona 85250 (US); GLIPA, Céline, 78480 Verneuil sur Seine (FR); RAJAGOPALAN, Pascal, 93600 Aulnay-sous-bois (FR); MABIC, Stéphane, 28260 Rouvres (FR)
(74) Representative: Loveless, Ian Mark
(86) International application number: PCT/US2008/059467
(87) International publication number: WO 2009/123645

(56) References cited:
- DE-A1- 4 424 355
- US-A1- 2007 278 096
- US-B1- 6 682 647
- US-B1- 6 790 341
- US-B1- 6 790 341
- US-B1- 6 878 255
- FARZANEH SHEMIRANI ET AL: "Simultaneous determination of traces of cadmium and zinc by adsorptive stripping voltammetry", CANADIAN JOURNAL OF ANALYTICAL SCIENCES AND SPECTROSCOPY, A, MORIN HEIGHTS, vol. 50, no. 4, 1 January 2005 (2005-01-01), pages 175-181, XP008141890, ISSN: 1205-6685
- UHLIG A ET AL: "Chip-array electrodes for simultaneous stripping analysis of trace metals", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 25, no. 1-3, 1 April 1995 (1995-04-01), pages 899-903, XP026903967, ISSN: 0925-4005, DOI: 10.1016/0925-4005(95)85198-4 [retrieved on 1995-04-01]
- XIE X ET AL: "Development of an ultramicroelectrode arrays (UMEAs) sensor for trace heavy metal measurement in water", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 97, no. 2-3, 1 February 2004 (2004-02-01), pages 168-173, XP004486614, ISSN: 0925-4005, DOI: 10.1016/J.SNB.2003.08.012
- PAESCHKE M ET AL: "VOLTAMMETRIC MULTICHANNEL MEASUREMENTS USING SILICON FABRICATED MICROELECTRODE ARRAYS", ELECTROANALYSIS, VHC PUBLISHERS, INC, US, vol. 8, no. 10, 1 January 1996 (1996-01-01), pages 1-08, XP009008415, ISSN: 1040-0397, DOI: 10.1002/ELAN.1140081008
- SHEMIRANI, FARZANEH ET AL.: 'Simultaneous determination of traces of cadmium and zinc by adsorptive stripping voltammetry.' CANADIAN JOURNAL OF ANALYTICAL SCIENCES AND SPECTROSCOPY vol. 50, no. 4, 2005, pages 175 - 181, XP008141890
- DUWENSEE, HEIKO ET AL.: 'Adsorptive stripping voltammetric detection of daunomycin at mercury and bismuth alloy electrodes.' INTERNATIONAL JOURNAL OF ELECTROCHEMICAL SCIENCE vol. 2, 2007, pages 498 - 507, XP008141895

## Description

### BACKGROUND

This application relates to electrochemical detection of materials. Detection of contaminants such as heavy mental ions in liquid is important in testing and protecting water supplies such as rivers and streams. Detection techniques based on chemiluminescence require presence of a catalyst enzyme to generate a flash of light. For example, Hach Eclox Rapid Response Water Test Kit is based on the reaction of luminol and an oxidant in the presence of a catalyst enzymehorseradish peroxidase (HRP). The reaction produces a flash of light which can be detected when an enhancer is added.

Examples for simultaneous voltammetric detection of multiple heavy metal ions are shown in US2007/0278096 A1, US 6,790,341 B1, US 6,682,647 B1 and DE 44 24 355 A1.

### SUMMARY

Techniques, systems and apparatus are disclosed for electrochemical detection of materials. In particular, trace levels of heavy metals ions, such as zinc (Zn), lead (Pb), cadmium (Cd), copper (Cu), mercury (Hg), arsenic (As), etc. can be detected using electrochemical detections.

In one aspect, detecting trace levels of metal ions includes a method according to any of claims 1-8.

Implementations can optionally include one or more of the following features. Applying the voltage can include applying an accumulation potential followed by a scanning potential. Applying a scanning potential can include applying a scanning potential having at least one of a 25 millivolt (mV) amplitude, a step potential of 4 millivolt (mV), and a frequency of 25 hertz. The voltage can be applied to the sensing electrode while enabling the sample liquid to flow across the sensing electrode. In addition, the voltage can be applied to another sensing electrode while another sample liquid flows across the other sensing electrode. The current signal is periodically measured to simultaneously detect a presence of two or more metal ions that includes two or more of zinc (Zn), lead (Pb), cadmium (Cd), copper (Cu), mercury (Hg) and arsenic (As). Applying the voltage can include applying the voltage to the sensing electrode that comprises at least one of bismuth, gold, carbon and mercury. Also, applying the voltage can include applying the voltage on the sensing electrode that comprises a screen printed electrode. Measuring the current signal can include measuring a voltammetric signal. Measuring the voltammetric signal can include using a stripping voltammeter to measure the voltammetric signal. Measuring the voltammetric signal using a stripping voltammeter can include using a disposable stripping voltammeter to measure the voltammetric signal. Further, the sample liquid can be pretreated with a buffer before enabling the sample liquid to flow through the container.

In another aspect, a system for detecting trace levels of heavy metal ions includes a detection device and a data processing device. The detection device includes a container to at least partially hold a sample liquid in the container with the sample liquid including one or more metal complexes. The detection device also includes an electrically conductive member that is at least partially submerged in the sample liquid. The container, the solution and the electrically conductive member are configured to measure a voltammetric signal corresponding to each metal complex. The electrical conductive member includes two or more sensing electrodes electrically connected in series, and a reference electrode coupled to the serially connected sensing electrodes and shared among the sensing electrodes. The data processing device is configured to identify in parallel two or more metal ions associated with the metal complexes based on the measured voltammetric signal. The container is shaped to form two or more channels that enable the sample liquid to flow through the container and across one of the sensing electrodes and another sample liquid to flow through the container and across another one of the sensing electrodes.

Implementations can optionally include one or more of the following features. The electrically conductive member can include at least one of bismuth, gold, carbon or mercury. The electrically conductive member can include a screen printed electrode. The detection device can be configured as a stripping voltammeter. The stripping voltammeter can include a disposable stripping voltammeter. Also, the detection device can be configured to measure voltammetric signal by applying an accumulation potential followed by a scanning potential. The system can also include a sample inlet and a sample outlet connected to the container to enable the sample liquid to flow through the detection device. The container is shaped to form a flow channel that enables the sample liquid to flow through the container and across the electrically conductive member. In addition, the data processing device is configured to periodically measure the voltammetric signal while the sample liquid flows across the electrically conductive member. Also, the data processing device is configured to periodically measure the voltammetric signal while the sample liquid flows across one of the sensing electrodes and the other sample liquid flows across the other sensing electrode.

The subject matter described in this specification potentially can provide one or more of the following advantages. The techniques and systems as described in this specification can be implemented as a miniaturized electrochemical analyzer to minimize or reduce costs. The miniaturized electrochemical analyzer can further reduce costs by implementing screen printed electrodes with integrated auxiliary and reference electrodes. The screen printed electrodes can be disposable for easy replacement. Also, the electrochemical analyzer can use multiple screen printed electrodes to simultaneously detect multiple heavy metal ions. To detect multiple heavy metal ions, the electrochemical analyzer can use analytical techniques such as stripping voltammetry. The miniaturized electrochemical analyzer can detect trace levels of the metal ions in the ppt range (ng/L). Further, the systems and techniques as described in this specification can provide on-line measurements of voltammetric signals.

The subject matter described in this specification can be implemented as electrochemical methods or systems for detecting the presence of materials such as, zinc, lead, cadmium, copper, mercury, arsenic, etc.

### BRIEF DESCRIPTION OF THE DRAWINGS

FIG. 1a is a block diagram showing an open configuration of a device for detecting various heavy metal ions.
FIG. 1b is a block diagram showing a closed configuration of a device for detecting various heavy metal ions.
FIG. 2 is a block diagram showing an example sample-to-electrode interface.
FIG. 3 is a process flow diagram an example process for detecting multiple heavy metal ions.
FIG. 4 shows example current-voltage relationships for different heavy metal ions.
FIG. 5 shows example calibration plots (current-concentration dependence) for different heavy metal ions.
FIG. 6 shows square wave absorptive stripping voltammetric signals for trace levels of various heavy metal ions.
FIG. 7 is a system for detecting multiple heavy metal ions using electrochemical detection.
FIG. 8 shows an example screen printed electrode that includes multiple working electrodes.

Like reference symbols and designations in the various drawings indicate like elements.

### Detailed Description

Techniques and systems are disclosed for detecting materials in liquid, such as water. In particular, an electrochemical detection device is provided to detect trace levels of heavy metals ions, such as zinc, lead, cadmium, copper, mercury, arsenic, etc. in water. The electrochemical detection device can use analytical techniques such as stripping voltammetry, for example.

Stripping voltammetry can be used as an analytical tool for detecting heavy metal ions in a variety of aqueous samples. Stripping voltammetry can be implemented in various configurations, such as deposition stripping voltammetry and adsorptive stripping voltammetry. Example techniques, systems and apparatus described in this specification use adsorptive stripping voltammetry. Adsorptive stripping analysis involves formation, adsorptive accumulation and reduction of a surface-active complex of a trace metal (e.g., Molybdenum (Mo)) with a ligand (e.g., chloranilate (CAA)). Thus, adsorptive stripping can be used to measure the concentration of a metal in presence of ligand (e.g., metal complex). An appropriate chelating agent or ligand may be employed.

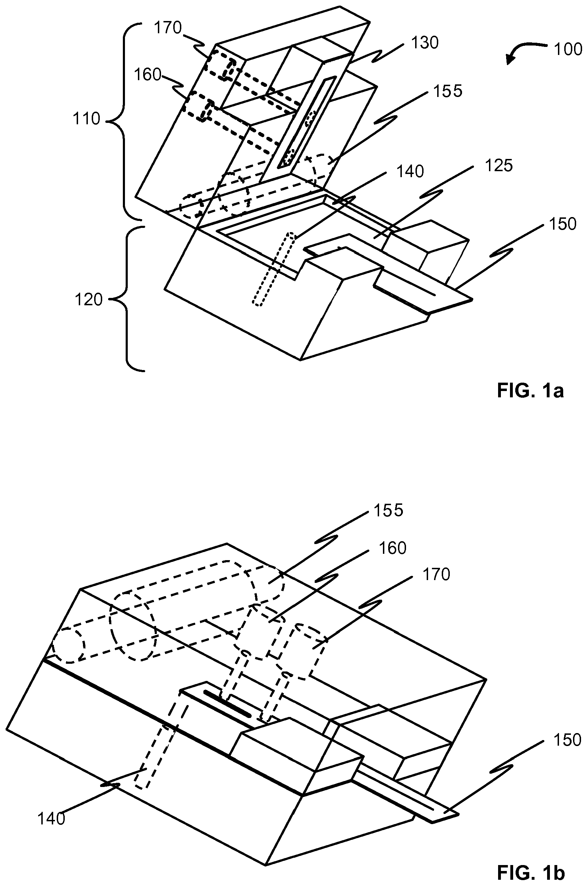

FIG. 1a shows an example device 100 for detecting trace levels of heavy metal ions in liquid. The device 100 can detect heavy metal ions, such as zinc, lead, cadmium, copper, mercury, arsenic, etc. in liquid using an analytical process, such as adsorptive stripping voltammetry. The device 100 includes a reference electrode 140, a sensing or working electrode 150, and a housing or container 125 for at least partially holding liquid. When the device 100 is implemented as a flow-through device, only a portion of the target liquid is actually held in the container 125. In some implementations, the device 100 can optionally include an auxiliary reference (not shown).

The reference electrode 140 is a conductive member that is constructed to at least partially conduct electricity. The reference electrode 140 can be implemented using various conductive materials. For example, a modified silver wire provided as an electrode (e.g., silver/silver-chloride (Ag/AgCI) when coated with AgCI and immersed in the recording solution) may be used.

The sensing electrode 150 can be implemented using any one of a wide variety of carbonaceous electrodes, such as carbon paste electrodes, glassy carbon electrodes, bare carbon electrodes, reticulated carbon electrodes, etc. Other suitable conducting electrodes, such as metallic electrodes can be used. Metallic electrodes can include gold, mercury and bismuth electrodes, for example. Further, the sensing electrode 150 can include bismuth coated or plated conducting electrodes. Alternatively, conducting electrodes with bismuth included analyte solution can be used. Bismuth based electrodes may provide good conductivity and low toxicity.

In some implementations, the sensing electrode 150 can be coated or plated with a thin film that contains conductive materials, such as bismuth. The conductive material-coated sensing or working electrode 150 can be a carbon fiber or glassy carbon electrode. The sensing electrode 150 can be coated with any conductive material, such as a flat screen-printed carbon electrode. For example, the sensing electrode 150 can be implemented as a disposable screen printed electrode that includes an auxiliary electrode. The disposable screen printed electrode can include a sensing or working electrode constructed using bismuth, gold or carbon. Also, the screen printed electrode can include the reference 140 and auxiliary electrodes, typically, silver/silver chloride and platinum, respectfully. In operation, the example device 100 implemented as a flow cell can be dismantled and the screen printed working electrode 150 can be replaced when desired.

In some implementations, other electrode constructions may be used. For example, the electrodes as disclosed in U.S. Pat. No. 6,682,647 and U.S. Patent No. 5,292,423, which are incorporated by reference, can be implemented as the sensing electrode 150. Briefly, screen-printed electrodes, such as the ExacTech Blood Glucose Strips of Medisense Inc, can be implemented. These strips include working and reference electrodes, made of carbon for example, printed on a polyvinyl chloride (PVC) substrate with carbon contacts on the opposite side. One printed carbon contact serves as a substrate for the mercury film electrode since the original working-electrode target area is covered with enzyme/mediator layers. The printed electrode (Ag/AgCI) from another strip serves as reference during voltammetric detection. Potentiometric stripping can also be performed using conventional Ag/AgCI electrode of the TraceLab unit. In addition, a platinum wire auxiliary electrode can be used.

The working electrode 150 can be constructed by coating and/or plating with different forms of bismuth. For example, solid bismuth in particulate or granular form can be used. Similarly, bismuth salts can be used as the active electrode material. In some implementations, solid bismuth or bismuth salts are used in a paste, gel or polymeric material which is coated or plated on the sensing electrode 150.

Also, the device 100 can include various optional components. For example, the device 100 can optionally include an inlet port 160 and an outlet port 170 to enable operation as a flow-through cell. When implemented as a flow-through cell, a stream of liquid (e.g., water) can flow through the device 100 using the inlet port 160 and outlet port 170. The presence of target heavy metal ions, such as zinc, lead, cadmium, copper, mercury, arsenic, etc. in the liquid flowing through the device can be continuously detected.

Alternatively, the device 100 can be operated in a batch mode to retain a discrete sample in the housing 125 without letting the liquid flow through. For example, a preset volume of the sample liquid can be deposited into the inlet port 160 and held in the housing 125 to detect the presence of one or more target heavy metal ions, such as zinc, lead, cadmium, copper, mercury, arsenic, etc.

In the open configuration, FIG. 1a shows a sealing member 130 made of rubber or other suitable sealing material designed to provide an air and/or water tight seal between a first member 110 and the second member 120. The first member 110 includes the inlet port 160, the outlet port 170 and the sealing member 130. The second member 120 includes the reference electrode 140, the sensing electrode 150 and the liquid housing 125. The first member 110 can be the top portion of the device 100, and the second member 120 can be the bottom portion of the device 100. Also, the first member 110 can be connected to the second member 120 through a joining mechanism 155 such as a hinge, a ball and socket joint, a tab, a dovetail, etc.

FIG. 1b shows a closed configuration view of the example device 100. When the device 100 is closed and liquid flows through the inlet port 160, an electrical current path is provided across the reference electrode 140 and the screen printed sensing electrode 150.

In addition, the form factor of the device 100 can be modified to simplify the analytical process of detecting trace levels of heavy metal ions, such as zinc, lead, cadmium, copper, mercury, arsenic, etc. in liquid. For example, the device 100 can be designed to simplify sample preparation and accelerate analysis time. The device 100 can be designed to use stripping voltammetry in batch mode using a discrete sample or in flow through mode. Little or no interaction between the user and the device 100, other than introducing the sample liquid and/or standards are needed.

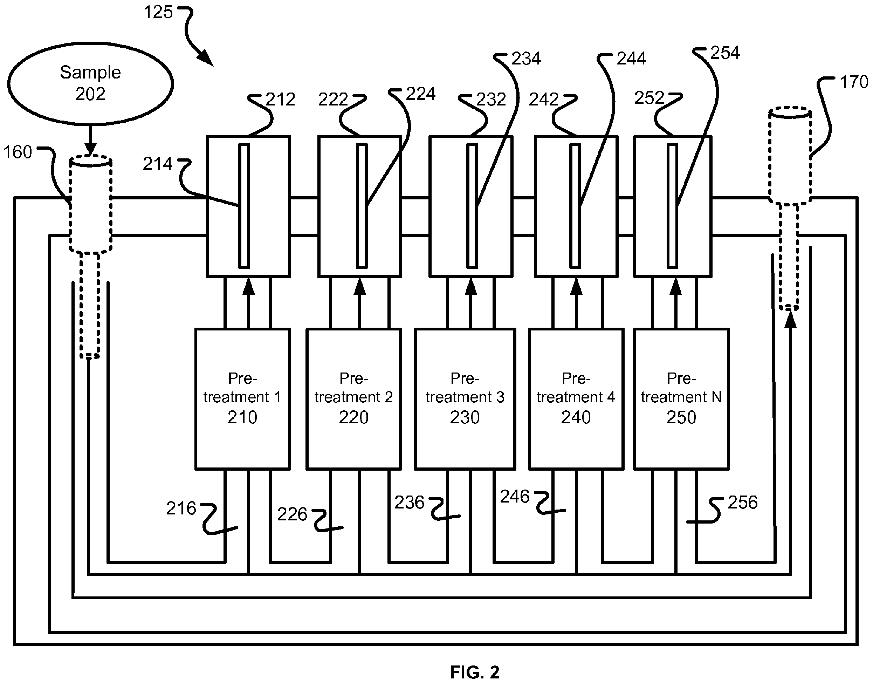

FIG. 2 is a block diagram illustrating an example interfaces for detecting trace levels of multiple heavy metal ions, such as zinc, lead, cadmium and copper. The housing or container 125 of the example device 100 can be structured to provide various sample-to-electrode interfaces. For example, FIG. 2 shows an example sample-to-electrode interface for using multiple screen printed electrodes 212, 222, 232, 242 and 252. These screen printed electrodes can be constructed substantially similar to the screen printed sensing electrode 150.

Each of the screen printed electrodes can be designed to monitor one or more specific heavy metal ions. By implementing multiple screen printed electrodes 212, 222, 232, 242 and 252, multiple heavy metal ions can be monitored simultaneously or in parallel. In the example shown in FIG. 2, the housing 125 is designed to enable the example device 100 to operate as a flow through cell that can include multiple different screen printed electrodes 212, 222, 232, 242 and 252 with each screen printed electrode having a different working electrode 214, 224, 234, 244 and 254, such as bismuth, gold, carbon, etc. Each working electrode can be constructed using the same or different materials selected from bismuth, gold, carbon, etc.

The example housing 125 can include two or more separate flow channels 216, 226, 236, 246 and 256 to simultaneously perform different sample pretreatments. Pretreatments performed can includes sample cleanup and processing, such as removal of interferences, acidification, etc. Through the separate flow channels 216, 226, 236, 246 and 256, the device 100 can monitor an increased number of heavy metal ions from a single sample 202. For example, the bismuth based working electrode described with respect to FIGS. 1a and 1b can be used in one flow channel (e.g. 216). In a separate flow channel (e.g. 226), a screen printed gold electrode that is selective for arsenic, selenium and mercury can be used.

The screen printed sensing electrodes 212, 222, 232, 242 and 252 can be constructed using a rapid and reproducible method. A carbon working electrode is screen printed, and a conductive material, such as gold, is selectively sputtered on carbon. The screen printed electrode can include both the reference and auxiliary electrodes. Each of the flow channels 216, 226, 236, 246 and 256 can house a separate screen printed electrode, conceivably with two different working electrodes, such as bismuth and gold that can be used simultaneously for their respective measurements.

Further, the different flow channels 216, 226, 236, 246 and 256 enables application of different pretreatment systems 210, 220, 230, 240 and 250 to each flow channel 216, 226, 236, 246 and 256. The pretreatment systems 210, 220, 230, 240 and 250 can provide a specific buffer and/or ligand to each flow channel to react with one or more heavy metal ions for parallel detection of multiple heavy metal ions.

The multiple electrochemical routes or flow channels for detecting multiple heavy metal ions simultaneously in water offers distinct advantages of speed, miniaturization and sensitivity. Thus, the device 100 can be implemented for field testing of various species of heavy metal ions. Also, by using bismuth or gold, the device 100 avoids the use of environmentally hazardous mercury.

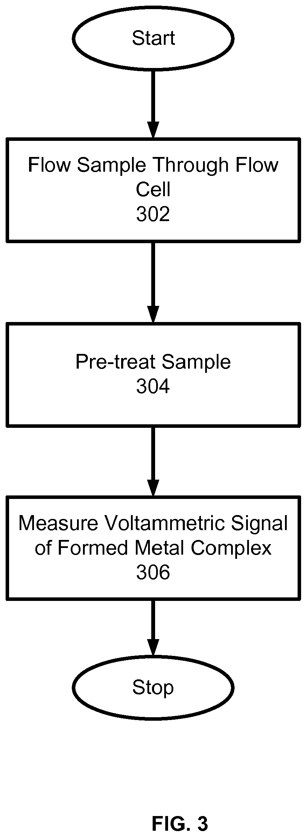

FIG. 3 shows an example process 300 for electrochemical detection of heavy metal ions in liquid. In particular, adsorptive stripping voltammetry can be used to measure and/or detect trace levels of heavy metal ions, such as zinc, lead, cadmium, copper, mercury, arsenic, etc. in water.

Obtained sample, such as sample water, is added 302 to the device 100 (via inlet port 160). The sample is separated into different flow channels 216, 226, 236, 246 and 256 and pre-treated 304 using one or more buffers and/or ligands. The pre-treatment can produce, for example, a metal complex, such as nickel-dimethylglyoximate (Ni-DMG), molybdenum-chloranilate (Mo-CAA), iron-catechol (Fe-catechol), etc. Using absorptive stripping voltammetry (e.g., using square wave absorptive stripping voltammetry), the voltammetric signal of the metal complex is measured 304 in an acetate pH buffer or other suitable buffering solutions. The voltammetric signal is measured, in this and other implementations, using the different working electrode 214, 224, 234, 244 and 254 (e.g., bismuth, mercury, gold, etc.) in each flow channel 216, 226, 236, 246 and 256.

Some heavy metals may not form complexes and thus do not require complexing agents. Examples of this heavy metal include lead (Pb), cadmium (Cd), mercury (Hg) and copper (Cu). In these types of heavy metals electrolytic accumulation can be used without such complexing agents.

The metal complex is electroactive and can be detected by voltammetry using the sensing or working electrodes electrode 214, 224, 234, 244 and 254. An accumulation potential of predefined amplitude is applied to the metal complex in the buffer for a predetermined period of time, followed by potential scanning with predefined amplitude, a predefined step potential and a predefined frequency. For example, square-wave adsorptive stripping voltammograms of 50 µg/L zinc with bismuth film electrode (BFE) can be implemented. In this example, zinc is in a solution of supporting electrolyte, 0.1 M acetate buffer (pH 5.5). An accumulation potential of 0.50V is applied for about 60 seconds with stirring. The accumulation potential is followed by potential scanning with amplitude of 25 mV, step potential of 4 mV, and a frequency of 25 Hz.

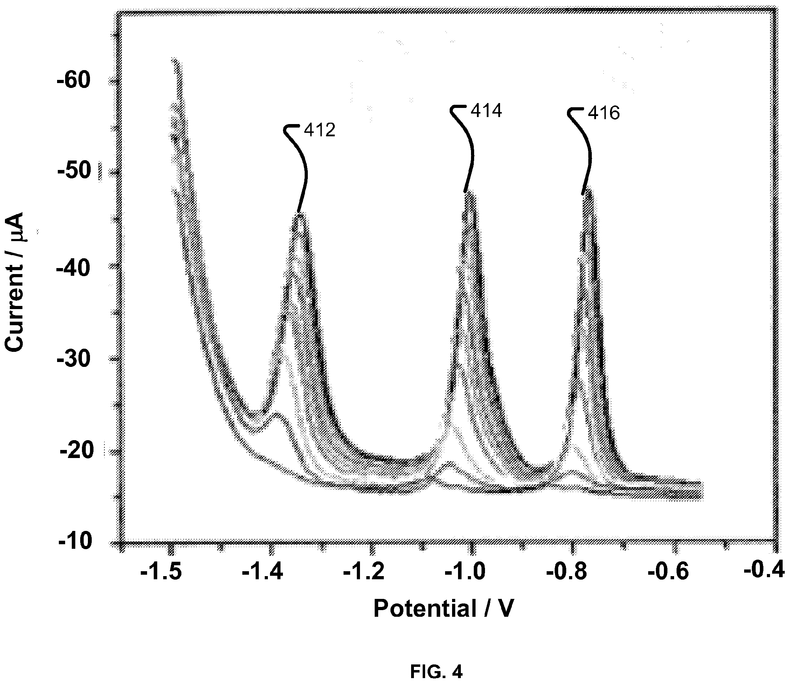

FIG. 4 shows an example simultaneous detection of zinc, lead and cadmium at various concentrations using the screen printed bismuth electrode 150 in the flow cell 100. While not shown in FIG. 4, other heavy metal ions such as copper can also be detected using the bismuth electrode 150. FIG. 4 shows the typical calibration peaks of zinc 412, cadmium 414 and lead 416 detected simultaneously using the bismuth electrode 150 in the flow cell 100. The calibration peaks for zinc 412, cadmium 414 and lead 416 as shown in FIG. 4 corresponds to a select concentration (e.g., 450 ppb) of Zn, Cd and Pb in 5 mM acetate buffer (pH 4.4). The peaks 412, 414 and 416 shown in FIG. 4 can be measured using a deposition time of 1 min with a sample liquid flowing through the flow cell 100 at a select flow rate (e.g., 1.0 mL/min). In addition, bismuth plating potential of -1.1V vs. Ag/AgCI can be used with bismuth plating time of 1200 s. The X-axis represents the potential used for the measurements, and the Y-axis represents the currents measured in µA.

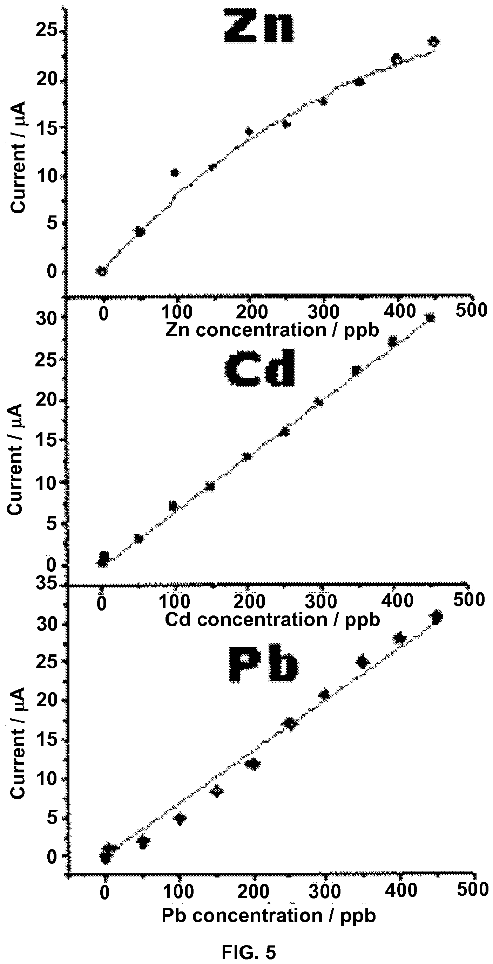

FIG. 5 shows the current-concentration relationship for Zn, Cd and Pb. The X-axis represents the concentration in parts per billion (ppb) of Zn, Cd and Pb. The Y-axis represents the measured current corresponding to the concentration of Zn, Cd and Pb. The current-concentration relationship appears to approach a linear relationship.

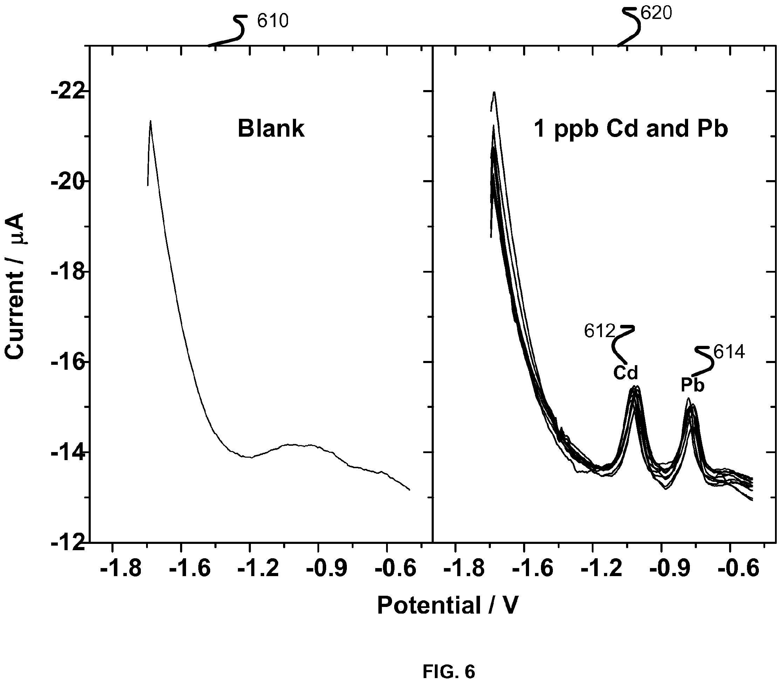

FIG. 6 shows the reproducibility of the screen printed electrodes 150, 212, 222, 232, 242 and 252 used in the flow-through cell 100 for 1 ppb of cadmium and lead. The left panel 610 shows current-voltage relationship for a blank without cadmium and lead. The right panel 620 shows the current-voltage relationship for 1 ppb of cadmium and lead. The current-voltage relationship for cadmium and lead shows clear current peaks for cadmium 612 and lead 614. The sizes of these current peak heights 612 and 614 are big enough to be visible even for the small 1 ppb concentration level. Thus, FIG. 6 shows that even lower trace levels can be detected under the same operating conditions.

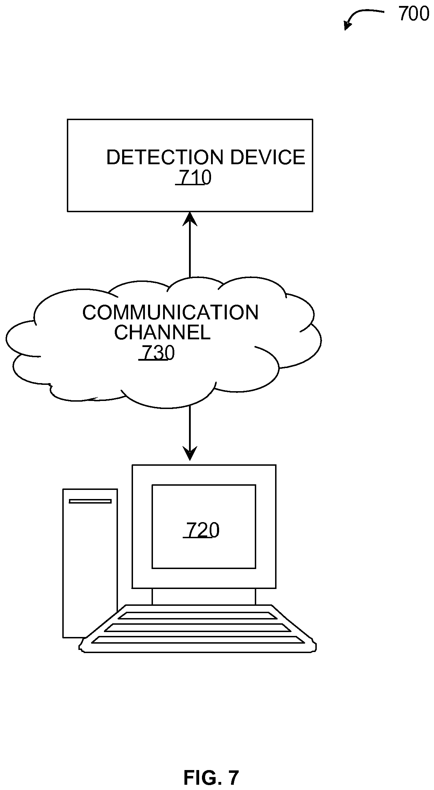

FIG. 7 shows an example system 700 for detecting heavy metal ions, such as zinc, lead, cadmium, copper, mercury, arsenic, etc. in liquid using electrochemical detection. The system 700 includes a detection device 710 (e.g., implemented in a dedicated flow cell design as shown in FIG. 1) coupled to a data processing device 720. The detection device 710 can be substantially as described with respect to FIG. 1. Alternatively, the detection device 710 can be implemented using other form factors.

The data processing device 720 can include various data processing devices for receiving data from the detection device 710 and processing the received data. For example, the data processing device 720 can include a personal computer (PC), a server computer, a portable computing device, etc. In addition, the data processing device 720 can include various components such as a central processing unit (CPU) designed to execute software programs. The execute software programs can include a program that performs data acquisition and analyses of the data received from the detection device 710. For example, the program can communicate an appropriate recording protocol for performing stripping voltammetry. The recording protocol can include the accumulation potential and the potential scanning with an amplitude, step potential, and a frequency.

The data processing device 720 receives the data from the detection device 710 through a bidirectional communication channel 730. The bidirectional communication channel 730 can include any one of wired or wireless connections. Wired connections can be compatible with Universal Serial Bus connections, FireWire connections, parallel connections, etc. Wireless connections can be compatible with Bluetooth, WiFi, Wimax, etc. In some implementations, the detection device can be placed at a remote location (e.g., in the field, such as a body of water) for testing a water source at the remote location. The data collected using the detection device 710 is transmitted to the data processing device 720 over a network such as wide area network (WAN), local area network (LAN), or the internet. In some implementations, the system 700 can be implemented as a stand alone system or as a part of a larger system such as network system.

In some implementations, the system 700 is implemented as a specific detection system that uses square wave absorptive stripping voltammetry. The detection system can use a bismuth electrode 150 to measure the voltammetric signal of the detected metal complex. The system 700 can be developed as a suitable and inexpensive system for electrochemical detection and measurement of heavy metal ions in pure and ultrapure water.

In addition, the system 700 can perform on-line voltammetric measurements using a dedicated flow cell 100. On-line measurements can be conducted by taking measurements in one or more flowing streams of sample liquid for continuous monitoring of target heavy metal ions **Alternatively,** the system 700 enables the user to inject a fixed volume of the untreated sample and the pre-treatment and detection processes can be performed on-line, without the user needing to touch the sample and potentially contaminate the sample.

Further, multiple electrodes 150, 212, 222, 232, 242 and 252 can be used with the system 700 to increase the number of heavy metal ions that can be detected in parallel. Such multi-detection system 700 is faster and more efficient than other processes that use one working electrode at any given time.

In some implementations, stripping voltammetry measurements obtained using the same screen printed electrodes 150, 212, 222, 232, 242 and 252 described with the flow-through device 100 can provide amplification of the detection sensitivity. Thus, trace levels of the heavy metal ions can be detected. The sensitivity enhancement can be provided using the concept of cells-in-series.

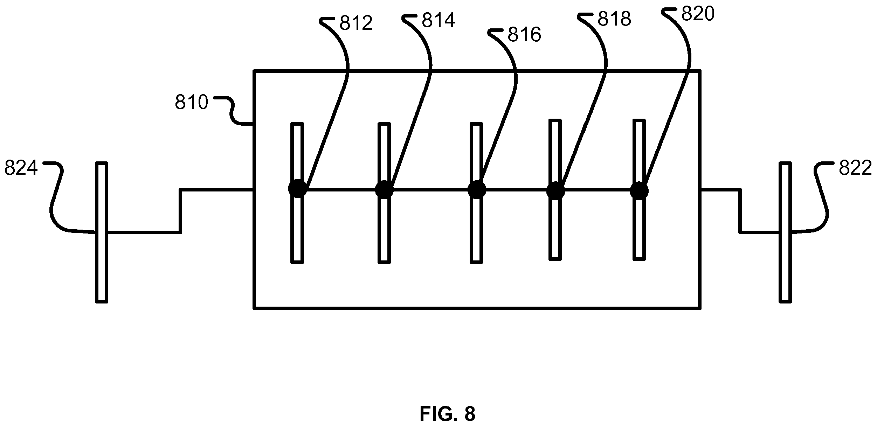

FIG. 8 shows an example representation of the cells-in-series concept. Multiple and identical working electrodes 812, 814, 816, 818 and 820 are disposed on a single screen printed electrode strip 810. The working electrodes 812, 814, 816, 818 and 820 are connected in series and share one reference 822 and one auxiliary electrode824. In some implementations, these multiple working electrodes 812, 814, 816, 818 and 820 can be a mix of different electrodes such as gold, bismuth, carbon, etc. In addition, these multiple electrodes can be operated under different measurement conditions, such as deposition potentials and window. Such combined structure can minimize the deposition time needed to achieve a lower detection limit.

Embodiments of the subject matter and the functional operations described in this specification can be implemented in digital electronic circuitry, or in computer software, firmware, or hardware, including the structures disclosed in this specification and their structural equivalents, or in combinations of one or more of them. Embodiments of the subject matter described in this specification can be implemented as one or more computer program products, i.e., one or more modules of computer program instructions encoded on a tangible program carrier for execution by, or to control the operation of, data processing apparatus. The tangible program carrier can be a propagated signal or a computer readable medium. The propagated signal is an artificially generated signal, e.g., a machine-generated electrical, optical, or electromagnetic signal that is generated to encode information for transmission to suitable receiver apparatus for execution by a computer. The computer readable medium can be a machine-readable storage device, a machine-readable storage substrate, a memory device, a composition of matter effecting a machine-readable propagated signal, or a combination of one or more of them.

The term "data processing apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, or a combination of one or more of them.

A computer program (also known as a program, software, software application, script, or code) can be written in any form of programming language, including compiled or interpreted languages, or declarative or procedural languages, and it can be deployed in any form, including as a stand alone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device.

Computer readable media suitable for storing computer program instructions and data include all forms of non volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, input from the user can be received in any form, including acoustic, speech, or tactile input.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described is this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), e.g., the Internet.

The computing system can include clients and servers. A client and server are generally remote from each other and typically interact through a communication network. The relationship of client and server arises by virtue of computer programs running on the respective computers and having a client-server relationship to each other.

Similarly, while operations are depicted in the drawings in a particular order, this should not be understood as requiring that such operations be performed in the particular order shown or in sequential order, or that all illustrated operations be performed, to achieve desirable results.

Only a few implementations and examples are described and other implementations, enhancements and variations can be made based on what is described and illustrated in this application. For example, implementations are not limited to the described metal complex, and metal complex based on other heavy metals and ligands are possible. Also, the recording protocol for adsorptive stripping voltammetry can be varied. For example, the recording protocol can be modified to change the amplitude of the accumulation potential; duration of accumulation potential applied; stirring or not stirring; amplitude of potential scanning; amplitude of the step potential; a frequency of the potentials applied.

## Claims

1. An electrochemical method for simultaneously detecting a presence of two or more metal ions comprising:
applying a voltage to two or more sensing electrodes (150, 214, 224, 234, 244, 254, 812, 814, 816, 818, 820) electrically connected in series while flowing a sample liquid that includes metal complexes across one of the two or more sensing electrodes via one of two or more channels;
flowing another sample liquid across another one of the sensing electrodes via another of the two or more channels;
in response to the applied voltage, periodically measuring a current signal associated with the metal complexes; and
processing the periodically measured current signal to simultaneously detect a presence of two or more metal ions associated with the metal complexes.

2. The method of claim 1, wherein applying the voltage comprises applying an accumulation potential followed by a scanning potential and/or wherein the scanning potential has at least one of 25 millivolt amplitude, a step potential of 4 millivolt, and a frequency of 25 Hertz.

3. The method of claim 1 or claim 2, wherein applying the voltage comprises:
applying the voltage to the sensing electrode while flowing the sample liquid across the sensing electrode; and
applying the voltage to another sensing electrode while flowing another sample liquid across the other sensing electrode.

4. The method of any one of claims 1 through 3, wherein the processing comprises processing the periodically measured current signal to simultaneously detect a presence of two or more metal ions that includes two or more of zinc (Zn), lead (Pb), cadmium (Cd), copper (Cu), mercury (Hg) and arsenic (As).

5. The method of any one of claims 1 through 4, wherein applying the voltage comprises applying the voltage to the sensing electrode that comprises at least one of bismuth, gold, carbon and mercury and/or wherein applying the voltage comprises applying the voltage on the sensing electrode that comprises a screen printed electrode.

6. The method of any one of claims 1 through 5, wherein measuring the current signal comprises measuring a voltammetric signal.

7. The method of any one of claims 1 through 6, wherein measuring the voltammetric signal comprises using a stripping voltammeter and/or a disposable stripping voltammeter to measure the voltammetric signal.

8. The method of any one of claims 1 through 7, further comprising pretreating the sample liquid with a buffer before flowing the sample liquid through the container.

9. A system comprising:
a detection device (100) comprising
a container (125) to at least partially hold a sample liquid in the container, the sample liquid including one or more metal complexes; and
an electrically conductive member that is at least partially submerged in the sample liquid, wherein the electrically conductive member is configured to measure a voltammetric signal corresponding to each metal complex, and wherein the electrical conductive member comprises
two or more sensing electrodes (150, 214,224,234,244,254, 812, 814, 816, 818, 280) electrically connected in series; and
a reference electrode (822) coupled to the serially connected sensing electrodes and shared among the sensing electrodes; and
a data processing device (720) configured to identify in parallel two or more metal ions associated with the metal complexes based on the measured voltammetric signal;
wherein the container is shaped to form two or more channels (216, 226, 236, 246, 256) that enable the sample liquid to flow through the container and across one of the sensing electrodes and another sample liquid to flow through the container and across another one of the sensing electrodes.

10. The system of claim 9, wherein the electrically conductive member comprises at least one of bismuth, gold, carbon or mercury and/or a screen printed electrode.

11. The system of claim 9 or claim 10, wherein the detection device is configured as a stripping voltammeter and/or wherein the stripping voltammeter comprises a disposable stripping voltammeter.

12. The system of any one of claims 9 through 11, wherein the detection device is configured to measure voltammetric signal by applying an accumulation potential followed by a scanning potential.

13. The system of any one of claims 9 though 12, further comprising a sample inlet and a sample outlet connected to the container to enable the sample liquid to flow through the detection device.

14. The system of any one of claims 9 through 13, wherein the container is shaped to form a flow channel that enables the sample liquid to flow through the container and across the electrically conductive member; and
wherein the data processing device is configured to periodically measure the voltammetric signal while the sample liquid flows across the electrically conductive member.

15. The system of any one of claims 9 through 14,
wherein the data processing device is configured to periodically measure the voltammetric signal while the sample liquid flows across one of the sensing electrodes and the other sample liquid flows across the other sensing electrode.

## Patentansprüche

1. Elektrochemisches Verfahren zum gleichzeitigen Erkennen einer Anwesenheit von zwei oder mehr Metallionen, umfassend:
Anlegen einer Spannung an zwei oder mehr Messelektroden (150, 214, 224, 234, 244, 254, 812, 814, 816, 818, 820), die elektrisch in Reihe geschaltet sind, während Fließenlassen einer Probenflüssigkeit, die Metallkomplexe aufweist, durch einen von zwei oder mehr Kanälen über eine der zwei oder mehr Messelektroden;
Fließenlassen einer weiteren Probenflüssigkeit durch einen anderen der zwei oder mehr Kanäle über eine andere der Messelektroden;
als Reaktion auf die angelegte Spannung periodisches Messen eines Stromsignals, das mit den Metallkomplexen assoziiert ist; und
Verarbeiten des periodisch gemessenen Stromsignals, um gleichzeitig eine Anwesenheit von zwei oder mehr Metallionen zu erkennen, die mit den Metallkomplexen assoziiert sind.

2. Verfahren nach Anspruch 1, wobei das Anlegen der Spannung das Anlegen eines Akkumulationspotentials gefolgt von einem Abtastpotential umfasst und/oder wobei das Abtastpotential wenigstens eines von einer 25-Millivolt-Amplitude, einem Stufenpotential von 4 Millivolt und einer Frequenz von 25 Hertz hat.

3. Verfahren nach Anspruch 1 oder Anspruch 2, wobei das Anlegen der Spannung Folgendes umfasst:
Anlegen der Spannung an die Messelektrode während Fließenlassen der Probenflüssigkeit über die Messelektrode; und
Anlegen der Spannung an eine andere Messelektrode während Fließenlassen einer anderen Probenflüssigkeit über die andere Messelektrode.

4. Verfahren nach einem der Ansprüche 1 bis 3, wobei das Verarbeiten das Verarbeiten des periodisch gemessenen Stromsignals zum gleichzeitigen Erkennen einer Anwesenheit von zwei oder mehr Metallionen umfasst, die zwei oder mehr von Zink (Zn), Blei (Pb), Cadmium (Cd), Kupfer (Cu), Quecksilber (Hg) und Arsen (As) aufweisen.

5. Verfahren nach einem der Ansprüche 1 bis 4, wobei das Anlegen der Spannung das Anlegen der Spannung an die Messelektrode umfasst, die wenigstens eines von Bismut, Gold, Kohlenstoff oder Quecksilber umfasst, und/oder wobei das Anlegen der Spannung das Anlegen der Spannung an die Messelektrode umfasst, die eine per Siebdruck gedruckte Elektrode umfasst.

6. Verfahren nach einem der Ansprüche 1 bis 5, wobei das Messen des Stromsignals das Messen eines voltammetrischen Signals umfasst.

7. Verfahren nach einem der Ansprüche 1 bis 6, wobei das Messen des voltammetrischen Signals das Verwenden eines Inversvoltammeters und/oder eines Wegwerf-Inversvoltammeters zum Messen des voltammetrischen Signals umfasst.

8. Verfahren nach einem der Ansprüche 1 bis 7, das ferner das Vorbehandeln der Probenflüssigkeit mit einem Puffer vor dem Fließenlassen der Probenflüssigkeit durch den Behälter umfasst.

9. System, umfassend:
eine Erkennungsvorrichtung (100), umfassend
einen Behälter (125), um eine Probenflüssigkeit wenigstens teilweise in dem Behälter aufzunehmen, wobei die Probenflüssigkeit ein oder mehr Metallkomplexe aufweist; und
ein elektrisch leitfähiges Element, das wenigstens teilweise in der Probenflüssigkeit eingetaucht ist, wobei das elektrisch leitfähige Element gestaltet ist, um ein jedem Metallkomplex entsprechendes voltammetrisches Signal zu messen, und wobei das elektrische leitfähige Element zwei oder mehr Messelektroden (150, 214, 224, 234, 244, 254, 812, 814, 816, 818, 820), die elektrisch in Reihe geschaltet sind, umfasst; und
eine Referenzelektrode (822), die mit den in Reihe geschalteten Messelektroden gekoppelt ist und unter den Messelektroden gemeinsam genutzt wird; und
eine Datenverarbeitungsvorrichtung (720), die zum parallelen Identifizieren von zwei oder mehr Metallionen, die mit den Metallkomplexen assoziiert sind, auf Basis des gemessenen voltammetrischen Signals konfiguriert ist;
wobei der Behälter so geformt ist, dass er zwei oder mehr Kanäle (216, 226, 236, 246, 256) bildet, die es ermöglichen, dass die Probenflüssigkeit durch den Behälter und über eine der Messelektroden fließt und eine andere Probenflüssigkeit durch den Behälter und über eine andere der Messelektroden fließt.

10. System nach Anspruch 9, wobei das elektrisch leitfähige Element wenigstens eines von Bismut, Gold, Kohlenstoff oder Quecksilber und/oder eine per Siebdruck gedruckte Elektrode umfasst.

11. System nach Anspruch 9 oder 10, wobei die Erkennungsvorrichtung als ein Inversvoltammeter konfiguriert ist und/oder wobei das Inversvoltammeter ein Wegwerf-Inversvoltammeter umfasst.

12. System nach einem der Ansprüche 9 bis 11, wobei die Erkennungsvorrichtung zum Messen eines voltammetrischen Signals durch Anlegen eines Akkumulationspotentials gefolgt von einem Abtastpotential konfiguriert ist.

13. System nach einem der Ansprüche 9 bis 12, das ferner einen Probeneinlass und einen Probenauslass umfasst, die mit dem Behälter verbunden sind, um zu ermöglichen, dass die Probenflüssigkeit durch die Erkennungsvorrichtung fließt.

14. System nach einem der Ansprüche 9 bis 13, wobei der Behälter geformt ist, um einen Durchflusskanal zu bilden, der es ermöglicht, dass die Probenflüssigkeit durch den Behälter und über das elektrisch leitfähige Element fließt; und
wobei die Datenverarbeitungsvorrichtung zum periodischen Messen des voltammetrischen Signals konfiguriert ist, während die Probenflüssigkeit über das elektrisch leitfähige Element fließt.

15. System nach einem der Ansprüche 9 bis 14, wobei die Datenverarbeitungsvorrichtung das voltammetrische Signal periodisch misst, während die Probenflüssigkeit über eine der Messelektroden fließt und die andere Probenflüssigkeit über die andere Messelektrode fließt.

## Revendications

1. Procédé électrochimique pour détecter simultanément une présence de deux ions métalliques ou plus, comprenant :
appliquer une tension à deux électrodes de détection ou plus (150, 214, 224, 234, 244, 254, 812, 814, 816, 818, 820) connectées électriquement en série tout en faisant couler un liquide échantillon qui comprend des complexes de métaux en travers l'une des deux électrodes de détection ou plus via l'un de deux canaux ou plus ;
faire couler un autre liquide échantillon en travers une autre des électrodes de détection via un autre des deux canaux ou plus ;
en réponse à la tension appliquée, mesurer périodiquement un signal de courant associé aux complexes de métaux ; et
traiter le signal de courant mesuré périodiquement pour détecter simultanément une présence de deux ions métalliques ou plus associés aux complexes de métaux.

2. Procédé selon la revendication 1, dans lequel appliquer la tension comprend appliquer un potentiel d'accumulation suivi par un potentiel de balayage et/ou dans lequel le potentiel de balayage a au moins l'un d'entre une amplitude de 25 millivolts, un potentiel d'échelon de 4 millivolts et une fréquence de 25 Hertz.

3. Procédé selon la revendication 1 ou la revendication 2, dans lequel appliquer la tension comprend :
appliquer la tension à l'électrode de détection tout en faisant couler le liquide échantillon en travers l'électrode de détection ; et
appliquer la tension à une autre électrode de détection tout en faisant couler un autre liquide échantillon en travers l'autre électrode de détection.

4. Procédé selon l'une quelconque des revendications 1 à 3, dans lequel le traitement comprend traiter le signal de courant mesuré périodiquement pour détecter simultanément une présence de deux ions métalliques ou plus qui comprennent deux ou plus de zinc (Zn), de plomb (Pb), de cadmium (Cd), de cuivre (Cu), de mercure (Hg) et d'arsenic (As).

5. Procédé selon l'une quelconque des revendications 1 à 4, dans lequel appliquer la tension comprend appliquer la tension à l'électrode de détection qui comprend au moins l'un d'entre bismuth, or, carbone et mercure et/ou dans lequel appliquer la tension comprend appliquer la tension à l'électrode de détection qui comprend une électrode sérigraphiée.

6. Procédé selon l'une quelconque des revendications 1 à 5, dans lequel mesurer le signal de courant comprend mesurer un signal voltampèrométrique.

7. Procédé selon l'une quelconque des revendications 1 à 6, dans lequel mesurer le signal voltampèrométrique comprend utiliser un multimètre de strippage et/ou un multimètre de strippage jetable pour mesurer le signal voltampèrométrique.

8. Procédé selon l'une quelconque des revendications 1 à 7, comprenant en outre prétraiter le liquide échantillon avec un tampon avant de faire couler le liquide échantillon à travers le récipient.

9. Système comprenant :
un dispositif de détection (100) comprenant
un récipient (125)
pour contenir au moins partiellement un liquide échantillon dans le récipient, le liquide échantillon comprenant un ou plusieurs complexes de métaux ; et
un membre électriquement conducteur qui est au moins partiellement submergé dans le liquide échantillon, dans lequel le membre électriquement conducteur est configuré pour mesurer un signal voltampèrométrique correspondant à chaque complexe de métaux, et dans lequel le membre électriquement conducteur comprend deux électrodes de détection ou plus (150, 214, 224, 234, 244, 254, 812, 814, 816, 818, 820) connectées électriquement en série ; et
une électrode de référence (822) couplée aux électrodes de détection couplées en série et partagée parmi les électrodes de détection ; et
un dispositif de traitement de données (720) configuré pour identifier en parallèle deux ions métalliques ou plus associés aux complexes de métaux sur la base du signal voltampèrométrique mesuré ;
où le récipient est façonné pour former deux canaux ou plus (216, 226, 236, 246, 256) qui permettent au liquide échantillon de couler à travers le récipient et en travers l'une des électrodes de détection et à un autre liquide échantillon de couler à travers le récipient et en travers d'une autre des électrodes de détection.

10. Système selon la revendication 9, dans lequel le membre électriquement conducteur comprend au moins l'une d'entre une électrode de bismuth, or, carbone ou mercure et/ou sérigraphiée.

11. Système selon la revendication 9 ou la revendication 10, dans lequel le dispositif de détection est configuré comme un multimètre de strippage et/ou dans lequel le multimètre de strippage comprend un multimètre de strippage jetable.

12. Système selon l'une quelconque des revendications 9 à 11, dans lequel le dispositif de détection est configuré pour mesurer un signal voltampèrométrique en appliquant un potentiel d'accumulation suivi par un potentiel de balayage.

13. Système selon l'une quelconque des revendications 9 à 12, comprenant en outre une admission d'échantillon et une sortie d'échantillon connectées au récipient pour permettre au liquide échantillon de couler à travers le dispositif de détection.

14. Système selon l'une quelconque des revendications 9 à 13, dans lequel le récipient est façonné pour former un canal d'écoulement qui permet au liquide échantillon de couler à travers le récipient et en travers le membre électriquement conducteur ; et
dans lequel le dispositif de traitement de données est configuré pour mesurer périodiquement le signal voltampèrométrique pendant que le liquide échantillon coule en travers le membre électriquement conducteur.

15. Système selon l'une quelconque des revendications 9 à 14, dans lequel le dispositif de traitement de données est configuré pour mesurer périodiquement le signal voltampèrométrique pendant que le liquide échantillon coule en travers l'une des électrodes de détection et que l'autre liquide échantillon coule en travers l'autre électrode de détection.
